(19) Europäisches Patentamt

European Patent Office

Office européen des brevets

(11) **EP 0 880 405 B1**

(12) **EUROPÄISCHE PATENTSCHRIFT**

(45) Veröffentlichungstag und Bekanntmachung des Hinweises auf die Patenterteilung:
**28.07.1999 Patentblatt 1999/30**

(21) Anmeldenummer: 97901555.9

(22) Anmeldetag: **20.01.1997**

(51) Int. Cl.$^6$: **B01J 19/00**

(86) Internationale Anmeldenummer:
**PCT/EP97/00236**

(87) Internationale Veröffentlichungsnummer:
**WO 97/26986 (31.07.1997 Gazette 1997/33)**

(54) **VORRICHTUNG UND VERFAHREN ZUM SYNTHETISIEREN VON MAKROMOLEKÜLEN**

DEVICE AND PROCESS FOR THE SYNTHETIZATION OF MACROMOLECULES

DISPOSITIF ET PROCEDE POUR SYNTHETISER DES MACROMOLECULES

(84) Benannte Vertragsstaaten:
**AT BE CH DE ES FR GB IT LI NL PT SE**

(30) Priorität: **23.01.1996 DE 19602159**

(43) Veröffentlichungstag der Anmeldung:
**02.12.1998 Patentblatt 1998/49**

(73) Patentinhaber:
• **Novartis AG**
**4058 Basel (CH)**
Benannte Vertragsstaaten:
**BE CH DE ES FR GB IT LI NL PT SE**
• **Novartis-Erfindungen Verwaltungsgesellschaft m.b.H.**
**1235 Wien (AT)**
Benannte Vertragsstaaten:
**AT**

(72) Erfinder:
• **BADER, Raoul**
**D-89075 Ulm (DE)**
• **HINZ, Michael**
**D-89134 Blaustein (DE)**
• **SELIGER, Hartmut**
**D-89275 Elchingen-Thalfingen (DE)**

(74) Vertreter: **Becker, Konrad et al**
**Novartis AG**
**Patent- und Markenabteilung CH**
**Lichtstrasse 35**
**4002 Basel (CH)**

(56) Entgegenhaltungen:
**WO-A-93/09668          WO-A-95/11748**
**US-A- 5 486 335**

**Beschreibung**

Die Erfindung betrifft eine Vorrichtung zum Synthetisieren von Makromolekülen insbesondere in sequenzhomologen Reihen mit wenigstens einem eine Anzahl von Reaktionskanälen aufweisenden Aufnahmeteil und mit einem Dichtabschnitte aufweisenden weiteren Aufnahmeteil, mit denen die Reaktionskanäle abdeckbar sind, wobei zwischen den Aufnahmeteilen ein in Synthesebereichen von Reaktionskanälen überdeckbares Trägermaterial in einer Anzahl von unterschiedlichen Anordnungen in bezug auf die Reaktionskanäle positionierbar ist.

Die Erfindung betrifft weiterhin ein Verfahren zum Synthetisieren von Makromolekülen insbesondere in sequenzhomologen Reihen, bei dem über eine Anzahl von Reaktionskanälen Reaktionslösungen in Synthesebereichen über ein Trägermaterial geleitet werden.

Eine derartige Vorrichtung und ein derartiges Verfahren sind aus der WO 95/11748 bekannt. Bei der gattungsgemässen Vorrichtung sind eine Anzahl von in ein Aufnahmeteil eingebrachten Reaktionskanälen vorgesehen, wobei ein Trägermaterial zwischen das die Reaktionskanäle aufweisende Aufnahmeteil und ein die Reaktionskanäle abdeckende Dichtabschnitte aufweisendes weiteres Aufnahmeteil einfügbar ist. Zur Synthese von Makromolekülen werden durch die Reaktionskanäle unterschiedliche Reagenzlösungen in einer ebenfalls unterschiedlichen sequentiellen Folge durchgeleitet.

Nach Ausführen einer Abfolge von ersten Syntheseschritten in einer ersten Positionierung, nach denen in jedem der von Reaktionskanälen überdeckten Synthesebereichen Makromoleküle mit ersten Sequenzen von Struktureinheiten vorliegen, werden die Reaktionskanäle in weiteren Positionierschritten derart beispielsweise um 90 Grad gedreht und eventuell anschliessend linear versetzt, dass sie in dieser neuen Position zuvor von anderen Reaktionskanälen überdeckte Synthesebereiche in Zellabschnitten überdecken. In einer Abfolge von weiteren Syntheseschritten sind den während der ersten Syntheseschritte in den Zellabschnitten gebildeten Makromolekülen weitere Sequenzabschnitte von Struktureinheiten anfügbar.

Zwar gestattet die gattungsgemässe Vorrichtung und das gattungsgemässe Verfahren die Bildung eines zweidimensionalen Feldes von Makromolekülen mit einer Kombination von Abschnitten mit unterschiedlichen Sequenzen, allerdings ist die Synthese von Makromolekülen unterschiedlicher Sequenzen aufgrund der grossen Anzahl von dazu benötigten, mit unterschiedlichen Reagenzlösungen zu beaufschlagenden Reaktionskanälen apparativ sehr aufwendig, der bei einer vertretbaren Anzahl von Positionierschritten und der Massgabe einer möglichst hohen Sequenzvarianz wegen der demzufolge verhältnismässig kleinen Zellabschnitte die quantitative Ausbeute verhältnismässig gering ist.

Der Erfindung liegt die Aufgabe zugrunde, eine Vorrichtung und ein Verfahren der eingangs genannten Art zu schaffen, das die Synthese von Makromolekülen unterschiedlicher Sequenz mit einer verhältnismässig einfachen Apparatur bei hoher Ausbeute gestattet.

Diese Aufgabe wird bei einer Vorrichtung der eingangs genannten Art erfindungsgemäss dadurch gelöst, dass Zwischenkanäle vorgesehen sind, über die eine Anzahl von Reaktionskanälen miteinander verbunden sind.

Diese Aufgabe wird bei einem Verfahren der eingangs genannten Art erfindungsgemäss dadurch gelöst, dass miteinander über Zwischenkanäle in Verbindung stehende Reaktionskanäle und das Trägermaterial bei verschiedenen Syntheseschritten in unterschiedlichen Relativpositionen Synthesebereiche überdeckend angeordnet werden.

Durch das relative Versetzen eines Trägermateriales zu einer Abfolge von über Zwischenkanäle miteinander verbundenen Reaktionskanälen um einen dem periodischen Abstand der Reaktionskanäle oder einem Vielfachen davon entsprechenden Weg sind in grossflächigen Synthesebereichen bei an den einzelnen Positionen aufeinanderfolgend durchgeführten Syntheseschritten Oligomere mit unterschiedlichen Sequenzen von Struktureinheiten gebildet, wobei bei jedem Syntheseschritt lediglich eine Reaktionslösung mit einer einzigen spezifischen Zusammensetzung durch die zusammenhängenden Reaktionskanäle geleitet ist. Durch den relativen Versatz der Reaktionskanäle auf beispielsweise unmittelbar benachbarte Synthesebereiche, die bei vorangehenden Syntheseschritten bereits von Reaktionslösungen anderer spezifischer Zusammensetzung beaufschlagt worden sind, lassen sich mit verhältnismässig wenigen Syntheseschritten mit die Reaktionskanäle in bezug auf das Trägermaterial versetzenden Positionierungen eine Vielzahl von Makromolekülen mit voneinander unterschiedlichen Sequenzen von einzelnen Struktureinheiten herstellen. So ist beispielsweise zum Aufbau von n Oligomeren mit einer gleichen Kettenlänge m lediglich die Anzahl m + n - 1 Syntheseschritten mit versetzendem Positionieren der Reaktionskanäle in bezug auf das Trägermaterial notwendig.

Die Reaktionskanäle sind vorzugsweise parallel äquidistant zueinander angeordnet. Dabei ist es für eine effektive Ausnutzung des Trägermateriales und im Hinblick auf einen kompakten Aufbau der Vorrichtung zweckmässig, dass der Zwischenabstand zwischen Reaktionskanälen kleiner als die Weite der Reaktionskanäle ist.

In Weiterbildungen eines ersten Typs sind die Reaktionskanäle rechtwinklig zu der Richtung des relativen Versatzes des Trägermateriales ausgerichtet. In Weiterbildungen eines zweiten Typs sind die Reaktionskanäle schräg zu der Richtung des relativen Versatzes des Trägermateriales angeordnet.

Bezüglich der Anordnung der Zwischenkanäle ist bei einer ersten Gruppe von Ausführungsbeispielen vorgesehen, dass sie in der Ebene der Reaktionskanäle angeordnet und an ihren dem Trägermaterial zugewandten Seiten offen sind. Diese Ausgestaltungen zeichnen sich durch eine einfache Herstellbarkeit der gesamten Kanalanordnung auf einer Seite eines Aufnahmeteiles beispielsweise durch Fräsen oder Abdruckformung aus.

In einer zweiten Gruppe von Ausgestaltungen sind Zwischenkanäle vorgesehen, die sich von dem Trägermaterial wegweisend als geschlossene Leitungen von einem Reaktionskanal zu einem weiteren Reaktionskanal erstrecken, so dass das Trägermaterial lediglich in von Reaktionskanälen überdeckten Synthesebereichen mit Reagenzlösungen beaufschlagt wird. Diese Anordnung zeichnet sich dadurch aus, dass in einer für eine spätere Weiterverarbeitung günstigen Weise lediglich in voneinander separierten Synthesebereichen Makromoleküle aufgebaut sind.

Es versteht sich, dass die Ausführungsbeispiele des ersten und zweiten Typs mit unterschiedlichen Ausrichtungen der Reaktionskanäle in Richtung des relativen Versatzes des Trägermateriales jeweils mit den verschiedenen Ausgestaltungen der Zwischenkanäle der vorangehend beschriebenen ersten und zweiten Gruppe von Ausführungsbeispielen kombinierbar sind.

Für eine rasche Synthese grösserer Mengen von Makromolekülen ist es zweckmässig, mehrere voneinander unabhängige Kanalanordnungen aus jeweils miteinander kommunizierenden Reaktionskanälen und Zwischenkanälen vorzusehen, die einseitig und/oder beidseitig des Trägermateriales angeordnet sind. Dadurch ergibt sich bei p Kanalanordnungen eine Anzahl von Kettenverlängerungsschritten mit $p(m + n - 1)$. Vorteilhafterweise sind bei einer beidseitigen Anordnung zwischen Reaktionskanälen eines Aufnahmeteiles Dichtabschnitte zum Abdecken von Reaktionskanälen eines anderen Aufnahmeteiles ausgebildet.

Zum Vermeiden von strömungsmässig toten Bereichen in den Reaktionskanälen und Zwischenkanälen ist es zweckmässig, dass sich die Zwischenkanäle an die Enden der Reaktionskanäle anschliessen und bei Kanten abgerundete Wandungen aufweisen.

Für eine gute Führung zum exakten Positionieren des zweckmässigerweise länglich ausgebildeten Trägermaterials ist es vorteilhaft, dass eines der auf das Probenmaterial aufzulegenden Aufnahmeteile eine längliche Führungsausnehmung und ein anderes Aufnahmeteil einen zu der Führungsvertiefung komplementären Führungsvorsprung aufweist, zwischen die das Trägermaterial einlegbar und bei Versatz zum Ausführen eines weiteren Syntheseschrittes durch die Ränder der Führungsvertiefung geführt kontrolliert relativ versetzbar ist.

Bei den Makromolekülen handelt es sich um sogenannte Biomakromoleküle, wie zum Beispiel Peptide oder Oligonukleotide, um abiologische, strukturell modifizierte Derivate derselben oder um synthetische Makromoleküle ohne biologischen Bezug.

Weitere zweckmässige Ausgestaltungen und Vorteile der Erfindung sind Gegenstand der Unteransprüche sowie der nachfolgenden Beschreibung von Ausführungsbeispielen unter Bezug auf die Figuren der Zeichnung. Es zeigen:

Fig. 1　　in einer perspektivischen Darstellung ein Ausführungsbeispiel einer Vorrichtung zum Synthetisieren von Makromolekülen mit einem zwischen Aufnahmeteilen angeordneten Trägermaterial,

Fig. 2　　in einer perspektivischen Darstellung das mit in einer Ebene mäanderförmig angeordneten Reaktionskanälen und Zwischenkanälen versehene Aufnahmeteil des Ausführungsbeispieles gemäss Fig. 1,

Fig. 3　　in einer Draufsicht die einem Trägermaterial zugewandte Seite des Aufnahmeteiles gemäss Fig. 2,

Fig. 4　　in einer perspektivischen Darstellung ein weiteres Ausführungsbeispiel einer Vorrichtung zum Synthetisieren von Makromolekülen mit Aufnahmeteilen und einem Ueberleitungsteil,

Fig. 5　　eine Draufsicht auf die einem Trägermaterial zugewandte Seite des Reaktionskanäle aufweisenden Aufnahmeteiles des Ausführungsbeispieles gemäss Fig. 4,

Fig. 6　　das Aufnahmeteil gemäss Fig. 5 in Draufsicht auf die dem Ueberleitungsteil gemäss Fig. 4 zugewandten Seite,

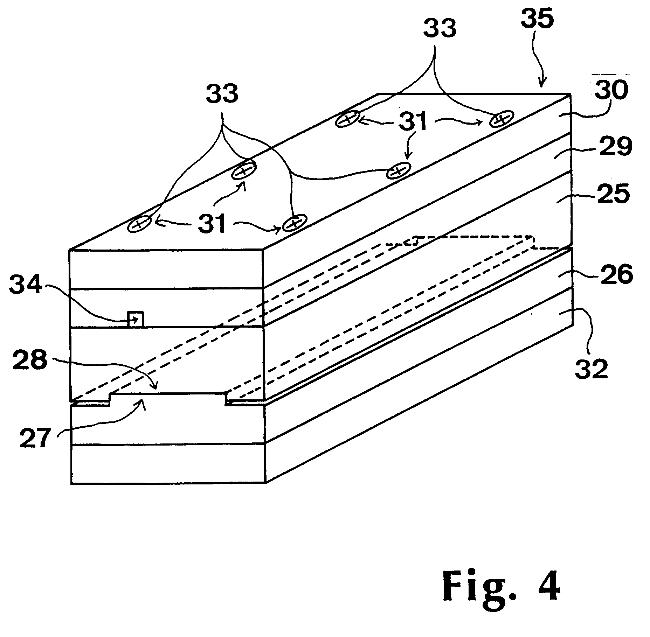

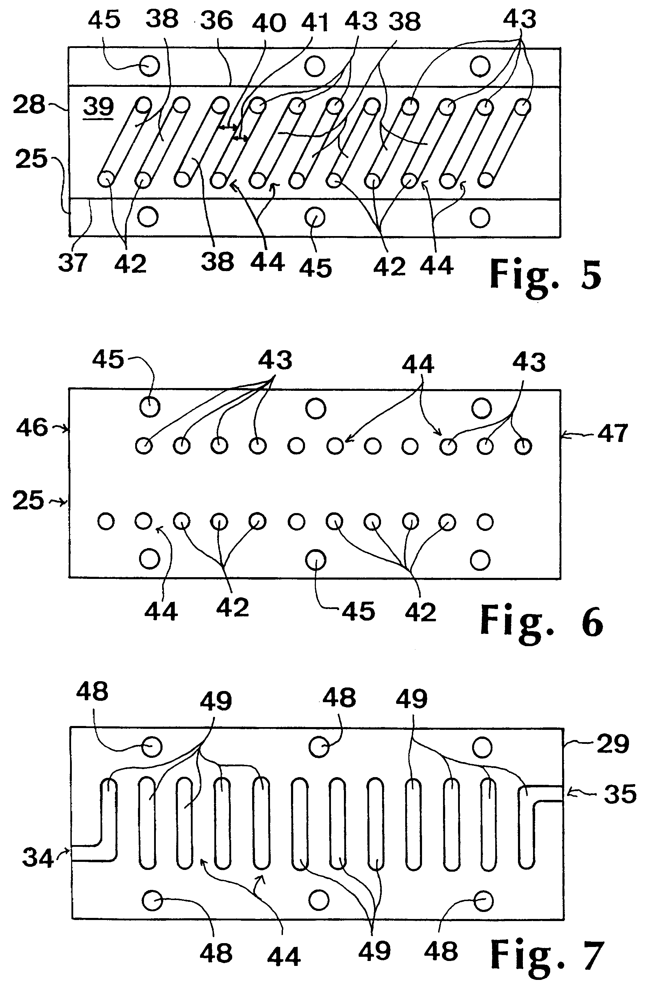

Fig .7　　das Ueberleitungsteil des Ausführungsbeispieles gemäss Fig. 4 in einer Draufsicht auf die dem in Fig. 5 und Fig. 6 dargestellten Aufnahmeteil zugewandte Seite,

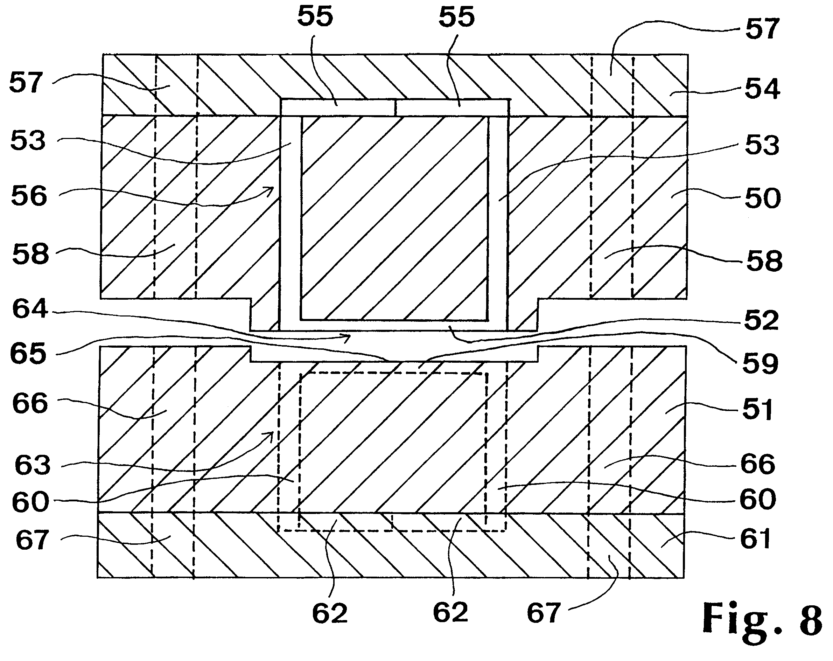

Fig. 8　　in einer Schnittansicht ein weiteres Ausführungsbeispiel einer Vorrichtung zum Synthetisieren von Makromolekülen, bei der in zwei sich gegenüberliegende Aufnahmeteile Reaktionskanäle eingebracht sind,

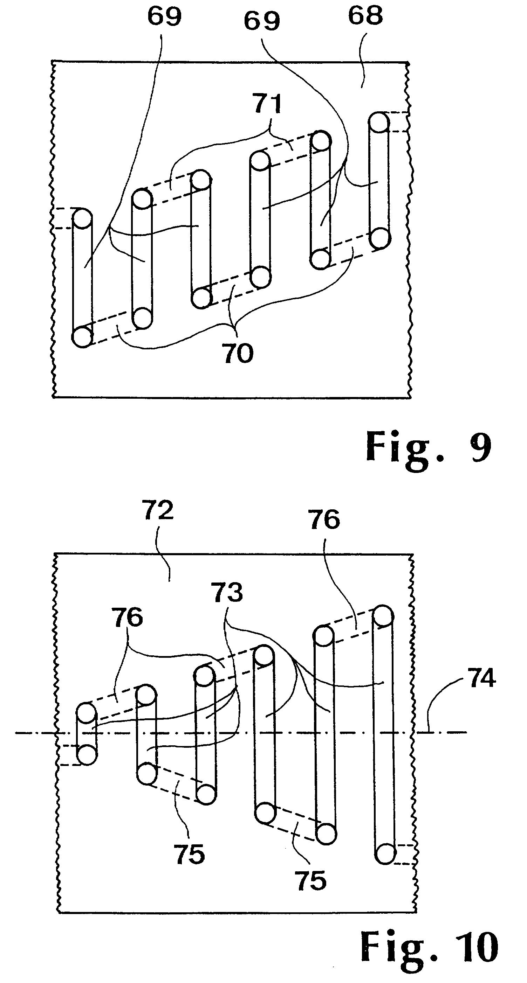

Fig. 9　　in einer Draufsicht die einem Trägermaterial zugewandte Seite eines Aufnahmeteiles, das mit in Längsrichtung zueinander versetzt angeordneten gleich langen Reaktionskanälen ausgestattet ist,

Fig. 10   in einer Draufsicht die einem Trägermaterial zugewandte Seite eines weiteren Aufnahmeteiles mit verschieden langen Reaktionskanälen,

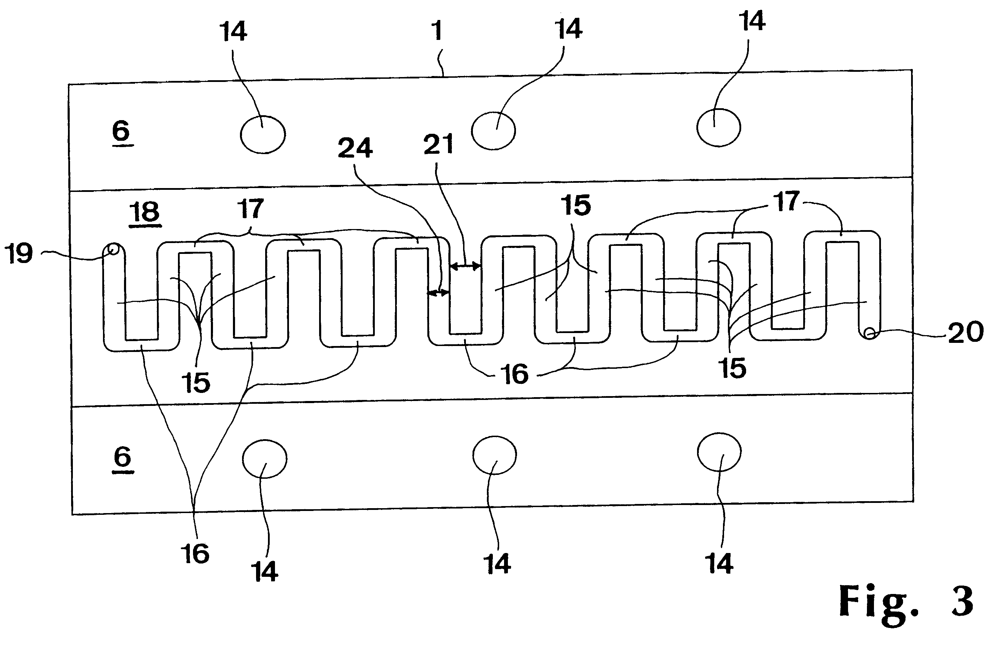

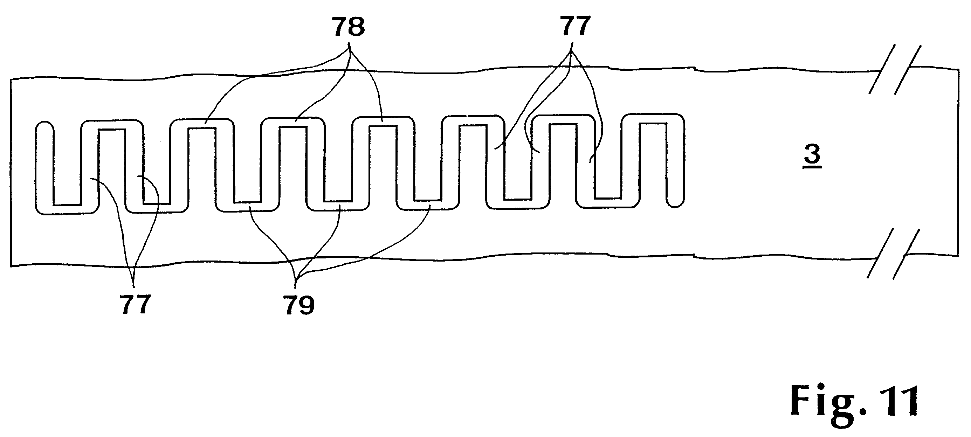

Fig. 11   in einer Draufsicht ein Trägermaterial mit den durch mäanderförmig in einer Ebene angeordneten Reaktionskanälen und Zwischenkanälen gemäss dem in Fig. 3 dargestellten Aufnahmeteil überdeckten Bereichen in einer ersten Position vor dem ersten Syntheseschritt,

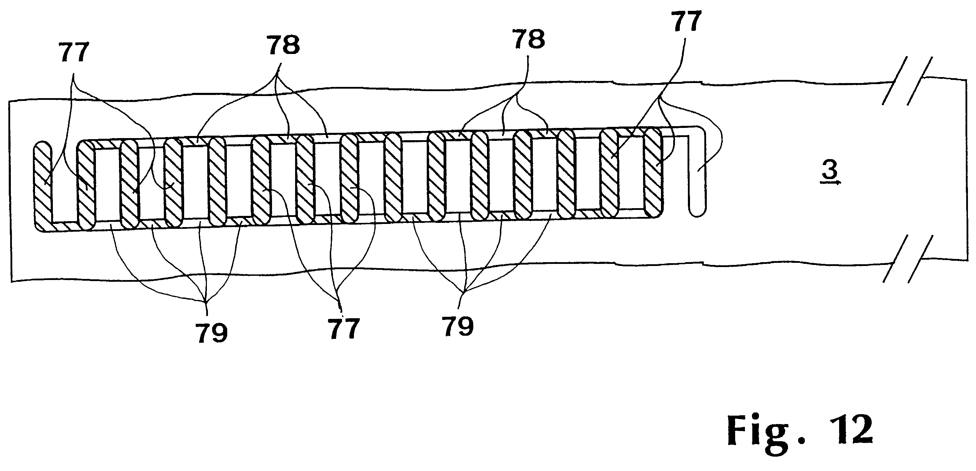

Fig. 12   das Trägermaterial gemäss Fig. 11 in einer zweiten Position vor dem zweiten Syntheseschritt,

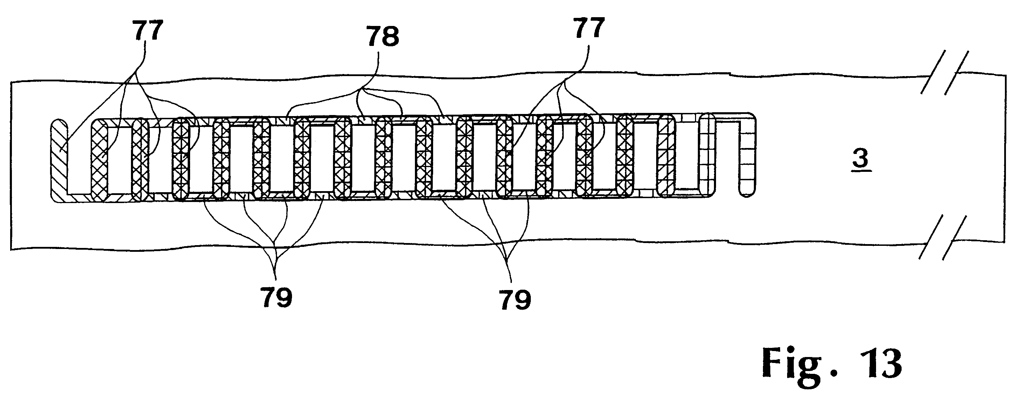

Fig. 13   das Trägermaterial gemäss Fig. 11 und Fig. 12 in einer vierten Position nach Ausführen des vierten Syntheseschrittes und

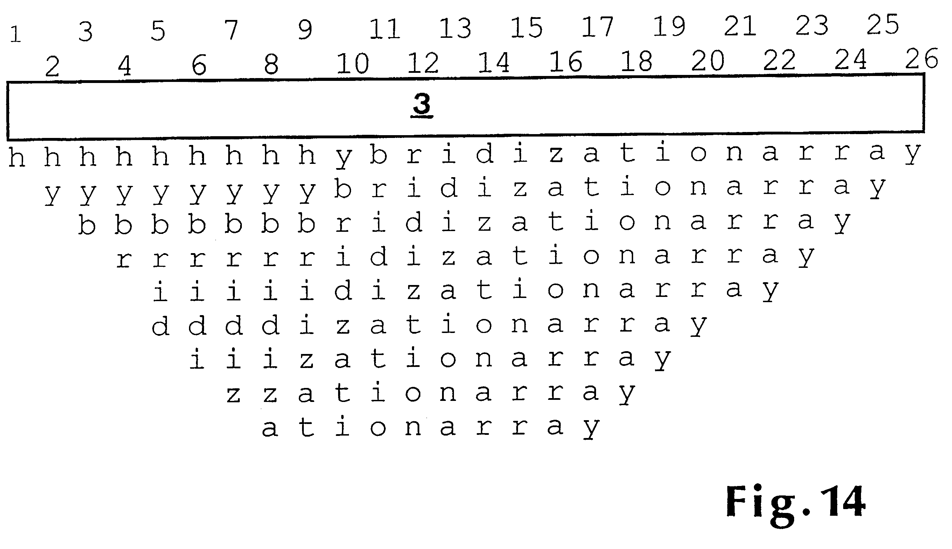

Fig. 14   eine beispielhafte Darstellung des Ergebnisses einer Synthese von Makromolekülen mit einem neun Reaktionskanäle aufweisenden Aufnahmeteil.

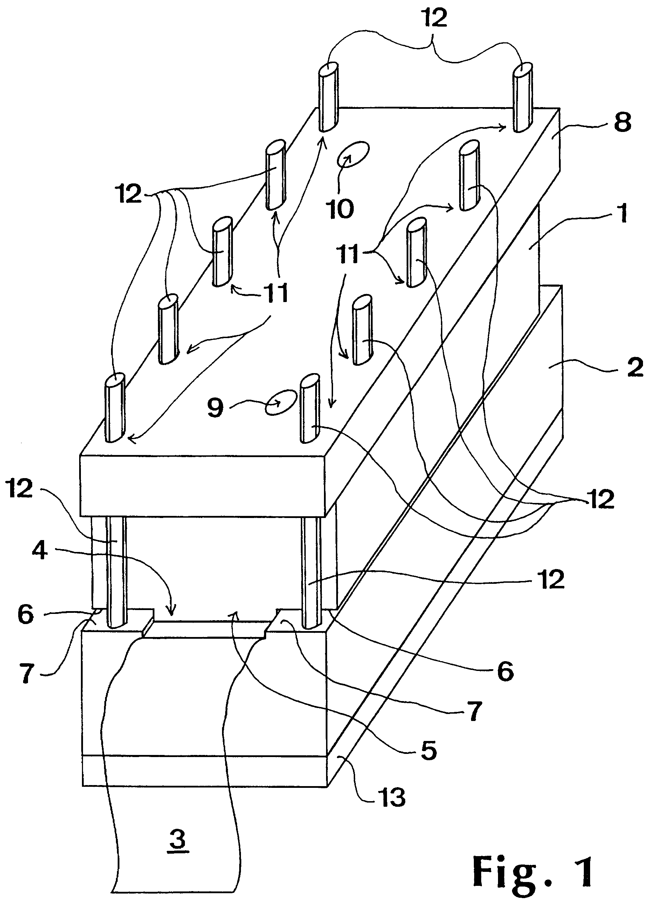

Fig. 1 zeigt in einer perspektivischen Darstellung ein Ausführungsbeispiel einer Vorrichtung zum Synthetisieren von Makromolekülen. Die in Fig. 1 dargestellte Vorrichtung verfügt über ein erstes Aufnahmeteil 1 und ein zweites Aufnahmeteil 2, die eine quaderförmige Gestalt aufweisen und zwischen denen eine längliche Trägerfolie 3 als Trägermaterial in einer von mehreren Anordnungen positioniert ist. Zweckmässigerweise ist die Trägerfolie 3 aus Polypropylen hergestellt. Bei dem in Fig. 1 dargestellten Ausführungsbeispiel verfügt das erste Aufnahmeteil 1 über einen sich in Längsrichtung erstreckenden Führungsvorsprung 4, der unter Einschluss der Trägerfolie 3 in eine komplementär ausgebildete Führungsausnehmung 5 des zweiten Aufnahmeteiles 2 einfügbar ist. Dabei ist es zweckmässig, dass der Ueberstand des Führungsvorsprunges 4 von beispielsweise 2 mm in bezug auf randseitige erste Seitenflächen 6 des ersten Aufnahmeteiles 1 grösser als die Tiefe der Führungsausnehmung 5 in bezug auf randseitige zweite Seitenflächen 7 des zweiten Aufnahmeteiles 2 von beispielsweise 1 mm ist, um ein Abdichten zu erzielen.

Vorteilhafterweise sind die Aufnahmeteile 1, 2 aus Polytetrafluorethylen (PTFE) oder einem anderen gegenüber den durchzuführenden Synthesen neutralen und plastisch verformbaren Kunststoff gefertigt. In abgewandelten, nicht dargestellten Ausführungsbeispielen sind die Aufnahmeteile 1, 2 aus Metall und elastische aus beispielsweise Silikon oder PTFE hergestellte Dichteelemente verwendet.

Auf die der Trägerfolie 3 abgewandten Seite des ersten Aufnahmeteiles 1 ist bei dem in Fig. 1 dargestellten Ausführungsbeispiel eine Deckplatte 8 aufgelegt, in die randseitig ein erster Synthesizeranschluss 9 und ein zweiter Synthesizeranschluss 10 eingebracht sind. An die Synthesizeranschlüsse 9, 10 ist als Synthesizer eine in Fig. 1 nicht dargestellte Einrichtung zur automatisierten Zufuhr und Abfuhr von Reagenzien enthaltenden Lösungsmitteln als Reagenzlösungen zum Durchführen von Syntheseschritten sowie von Spüllösungen zur Reinigung anschliessbar.

Weiterhin verfügt die Deckplatte 8 über eine Anzahl von Durchsteckausnehmungen 11, durch die Gewindestangen 12 durchsteckbar sind. Die Gewindestangen 12 durchqueren in entsprechenden Durchsteckausnehmungen randseitig die Aufnahmeteile 1, 2 und sind in eine auf der der Trägerfolie 3 abgewandten Seite des zweiten Aufnahmeteiles 2 angeordnete Grundplatte 13 einschraubbar, die mit entsprechenden Innengewinden versehen ist. Durch Aufschrauben von in Fig. 1 nicht dargestellten Muttern auf die über die Deckplatte 8 vorstehenden Enden der Gewindestangen 12 sind die Aufnahmeteile 1, 2 mit einstellbaren Pressdrücken aneinanderfügbar sowie zum relativen Positionieren der Trägerfolie 3 durch Lockern beziehungsweise Abschrauben der Muttern voneinander unter Lösen der Abdichtung separierbar.

Bei einer gegenüber dem in Fig. 1 dargestellten Ausführungsbeispiel abgeänderten Ausgestaltung sind anstatt der Gewindestangen und Muttern an der dem ersten Aufnahmeteil 1 abgewandten Seite der Deckplatte 8 aufliegende, an glattwandigen Durchsteckstangen angebrachte Kniehebel vorgesehen, die in besonders einfach zu handhabender Art und Weise das Anpressen und Lösen der Aufnahmeteile 1, 2 ermöglichen.

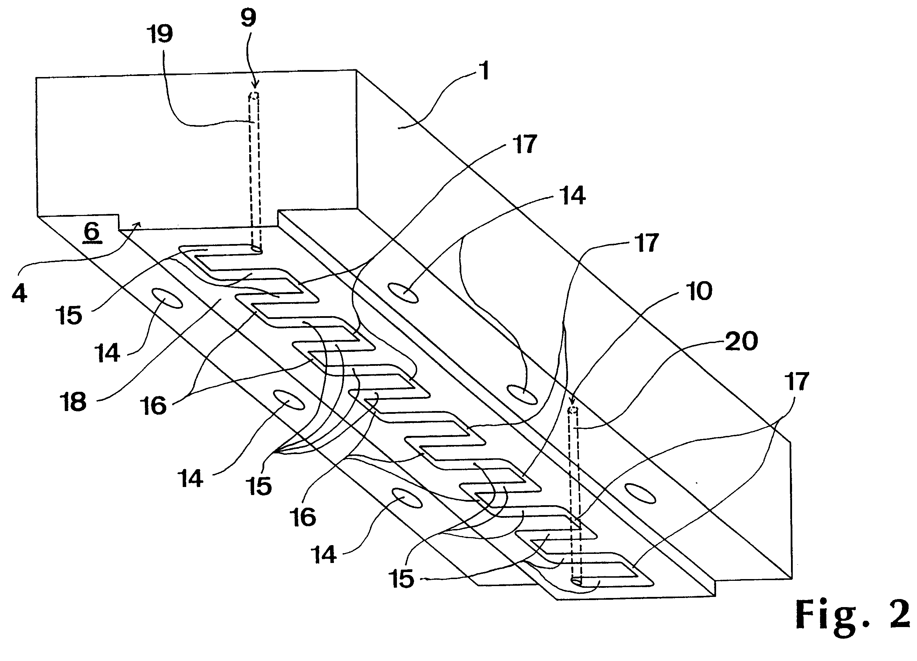

Fig. 2 zeigt in einer perspektivischen Darstellung das erste Aufnahmeteil 1 der anhand Fig. 1 erläuterten Vorrichtung in einer Ansicht auf die der Trägerfolie 3 zugewandten Seite, wobei lediglich einzelne in Verlängerung der Durchsteckausnehmungen 11 der Deckplatte 8 angeordnete Durchsteckausnehmungen 14 dargestellt und mit Bezugszeichen versehen sind. Das erste Aufnahmeteil 1 verfügt im Bereich des Führungsvorsprunges 4 über eine mäanderförmige Anordnung einer Anzahl von Reaktionskanälen 15 mit einer Länge von beispielsweise 10 mm und jeweils zwei benachbarte Reaktionskanäle 15 miteinander verbindenden Zwischenkanälen 16, 17, die bei dem ersten Aufnahmeteil 1 gemäss Fig. 2 in einer durch eine Trägerauflagefläche 18 des Führungsvorsprunges 4 gebildeten Ebene angeordnet und an ihrer der Trägerfolie 3 zugewandten Seite offen sind. Die Reaktionskanäle 15 und Zwischenkanäle 16, 17 sind beispielsweise 0,3 mm tief. Eine erste Gruppe von Zwischenkanälen 16 verbindet jeweils zwei benachbarte Reaktionskanäle 15 mit ihren gleichseitig angeordneten Enden, während eine zweite Gruppe von Zwischenkanälen 17 die jeweils anderen Enden von zwei benachbarten Reaktionskanälen 15 miteinander verbindet, so dass eine eben mäanderförmige Anordnung von Reaktionskanälen 15 mit einer alternierenden Folge von endseitig der Reaktionskanäle 15 verlaufenden Zwischenkanälen 16, 17 mit einer Breite von beispielsweise 1 mm gebildet ist.

Der erste Synthesizeranschluss 9 steht über eine erste Anschlussausnehmung 19 mit einem Durchmesser von beispielsweise 1 mm mit dem geschlossenen Ende des in der Darstellung gemäss Fig. 2 vorderen randseitigen Reaktionskanal 15 in Verbindung, während der in der Darstellung gemäss Fig. 2 rückseitig gelegene zweite Synthesizeranschluss 10 mit dem geschlossenen Ende des anderen randseitig angeordneten Reaktionskanales 15 über eine zweite Anschlussausnehmung 20 mit einem Durchmesser von beispielsweise 1 mm in Verbindung steht.

Fig. 3 zeigt das erste Aufnahmeteil 1 gemäss Fig. 1 und Fig. 2 in einer Draufsicht auf die der Trägerfolie 3 zugewandten Seite. Die Reaktionskanäle 15 sind parallel zueinander angeordnet und weisen einen jeweils gleichen Reaktionskanalzwischenabstand 21 von beispielsweise 3 mm auf. Die Reaktionskanäle 15 verlaufen im wesentlichen rechtwinklig zu den zueinander parallelen Randseiten 22, 23 des Führungsvorsprunges 4, entlang derer die Trägerfolie 3 in Längsrichtung versetzbar ist. Die Reaktionskanäle 15 weisen eine jeweils gleiche Reaktionskanalweite 24 von beispielsweise 2 mm auf. Beim Versetzen der Trägerfolie 3 relativ zu den Reaktionskanälen 15 in Längsrichtung um einen aus der Summe des Reaktionskanalzwischenabstandes 21 und der Reaktionskanalweite 24 entsprechenden Weg von beispielsweise 5 mm bei den oben angegebenen beispielhaften Massen oder einem ganzzahligen Vielfachen davon kommen eine Anzahl der Reaktionskanäle 15 bei einer vorangegangenen Position bereits von Reaktionskanälen 15 bedeckten Synthesebereichen zu liegen, während andere in Versatzrichtung vorderseitig gelegene Reaktionskanäle 15 erstmalig neue Synthesebereiche überdecken.

Die bei dem anhand Fig. 2 und Fig. 3 erläuterten Ausführungsbeispiel vorgesehenen Reaktionskanäle 15 sind gleich lang und mit ihren Enden fluchtend angeordnet, so dass die Zwischenkanäle 16 beziehungsweise die Zwischenkanäle 17 ebenfalls ineinander fluchten.

Im zusammengefügten Zustand der Auflageteile 1, 2 sind die Reaktionskanäle 15 sowie Zwischenkanäle 16, 17 durch auf der der Trägerauflagefläche 18 gegenüberliegenden Flachseite der Führungsausnehmung 5 ausgebildete Dichtabschnitte, die die offenen Seiten der Reaktionskanäle 15 und Zwischenkanäle 16, 17 bedecken, abgedichtet.

In einer gegenüber dem anhand Fig. 2 und Fig. 3 erläuterten Ausführungsbeispiel abgeänderten Ausgestaltung sind die Reaktionskanäle 15 in bezug auf die Randseiten des Führungsvorsprunges 4 mit jeweils gleichen Neigungswinkeln schiefwinklig mit jeweils fluchtenden Zwischenkanälen 16, 17 angeordnet. Diese Ausgestaltung zeichnet sich durch gegenüber einer rechtwinkligen Anordnung von Reaktionskanälen 15 grossflächigere Synthesebereiche bei gleichem Abstand von sich gegenüberliegenden Zwischenkanälen 16, 17 aus.

Fig. 4 zeigt in einer perspektivischen Darstellung ein weiteres Ausführungsbeispiel einer Vorrichtung zum Synthetisieren von Makromolekülen. Bei der in Fig. 4 dargestellten Vorrichtung sind ein erstes Aufnahmeteil 25 und ein zweites Aufnahmeteil 26 mit jeweils quaderförmiger Gestalt vorgesehen, die unter Eingriff eines sich mittig in Längsrichtung des zweiten Aufnahmeteiles 26 erstreckenden Führungsvorsprunges 27 in eine komplementär zu dem Führungsvorsprung 27 ausgebildete, in das erste Aufnahmeteil 25 eingebrachte Führungsausnehmung 28 einfügbar sind, wobei der Ueberstand des Führungsvorsprunges 27 grösser als die Tiefe der Führungsausnehmung 28 ist.

Auf der dem zweiten Aufnahmeteil 26 gegenüberliegenden Seite des ersten Aufnahmeteiles 25 ist ein Ueberleitungsteil 29 aufgelegt, das wiederum auf seiner dem ersten Aufnahmeteil 25 gegenüberliegenden Seite mit einer Deckplatte 30 belegt ist. In der Deckplatte 30 sind in Längsrichtung randseitig eingebrachte Durchsteckausnehmungen 31 vorgesehen, an die sich fluchtend jeweils durch das Ueberleitungsteil 29 sowie die Aufnahmeteile 25, 26 weitere Durchsteckausnehmungen erstrecken. An der dem ersten Aufnahmeteil 25 gegenüberliegenden Seite des zweiten Aufnahmeteiles 26 ist eine Grundplatte 32 angelegt, die über die Durchsteckausnehmungen abschliessende Gewinde verfügt. In der Darstellung gemäss Fig. 4 sind in diese Gewinde die Durchsteckausnehmungen durchquerende Befestigungsschrauben 33 zum Zusammenpressen der Aufnahmeteile 25, 26 eingeschraubt. In dem Ueberleitungsteil 29 sind weiterhin in der Darstellung gemäss Fig. 4 an der vorderen Stirnseite ein erster Synthesizeranschluss 34 und an der hinteren Stirnseite ein zweiter Synthesizeranschluss 35 vorgesehen.

Fig. 5 zeigt das erste Aufnahmeteil 25 gemäss Fig. 4 in einer Draufsicht auf die dem zweiten Aufnahmeteil 26 zugewandte Seite. In die Führungsvertiefung 28 sind in bezug auf deren sich in Längsrichtung erstreckenden parallelen Kantseiten 36, 37 schiefwinklig parallel äquidistant angeordnete Reaktionskanäle 38 eingebracht, die zu der dem zweiten Aufnahmeteil 26 zugewandten Trägerauflagefläche 39 offen sind. Der Reaktionskanalzwischenabstand 40 zwischen einander zugewandten Randseiten der Reaktionskanäle 38 sowie die Reaktionskanalweite 41 jeweils in Längsrichtung der Kantseiten 36, 37 legen entsprechend dem anhand Fig. 3 erläuterten Ausführungsbeispiel den Versatzweg eines zwischen die Aufnahmeteile 25, 26 eingebrachten Trägermateriales fest, um Synthesebereiche wiederholt exakt mit Reaktionskanälen 38 zu überdecken.

In den Endbereichen jeder der Reaktionskanäle 38 münden Schenkelausnehmungen 42, 43 von geschlossene Leitungen bildenden Zwischenkanälen 44. Die Schenkelausnehmungen 42, 43 queren das erste Aufnahmeteil 25 von der Trägerauflagefläche 39 auf die dem Ueberleitungsteil 29 zugewandte Seite. In dem in Fig. 5 dargestellten Ausführungsbeispiel sind die Schenkelausnehmungen 42, 43 durch rundliche Bohrungen mit einem der Breite der Reaktionskanäle 38 entsprechenden Durchmesser ausgeführt. Weiterhin sind in Fig. 5 die in das erste Aufnahmeteil 25 eingebrachten Durchsteckausnehmungen 45 dargestellt.

Fig. 6 zeigt das in Fig. 5 dargestellte erste Aufnahmeteil 25 in einer Draufsicht auf die dem Ueberleitungsteil 29 zugewandte Seite. Die sich durch das erste Aufnahmeteil 25 erstreckenden Schenkelausnehmungen 42, 43 sind in der Anordnung der Reaktionskanäle 38 gemäss Fig. 5 zum einen in einer ersten Gruppe von Schenkelausnehmungen 43 in Längsrichtung des ersten Aufnahmeteiles 25 miteinander fluchtend und in einer zweiten Gruppe von Schenkelausnehmungen 43 in gleichem Abstand zu der ersten Gruppe von Schenkelausnehmungen 42 in Längsrichtung des ersten Aufnahmeteiles 25 miteinander fluchtend angeordnet. Weiterhin liegen benachbarten Reaktionskanälen 38 zugeordnete Schenkelausnehmungen 42, 43 einander in Querrichtung parallel zu den Stirnseiten 46, 47 des ersten Aufnahmeteiles 25 gegenüber. Diese durch den Reaktionskanalzwischenabstand 40, die Reaktionskanalweite 41 sowie den Neigungswinkel der Reaktionskanäle 38 bedingte Anordnung der Schenkelausnehmungen 42, 43 führt zu einem besonders einfachen Einbringen von Bohrungen zum Herstellen der Schenkelausnehmungen 42, 43.

Fig. 7 zeigt eine Draufsicht auf die dem ersten Aufnahmeteil 25 zugewandte Seite des Ueberleitungsteiles 29. In diese Seite des Ueberleitungsteiles 29 sind neben den Durchsteckausnehmungen 48 sich zwischen in Querrichtung des ersten Aufnahmeteiles 25 gegenüberliegenden Schenkelausnehmungen 42, 43 erstreckende Ueberleitungsausnehmungen 49 eingebracht, die auf ihrer dem ersten Aufnahmeteil 25 zugewandten Seite offen sind und zusammen mit den Schenkelausnehmungen 42, 43 die Zwischenkanäle 44 bilden. Die randseitigen Ueberleitungsausnehmungen 49 setzen sich mit einem Längsabschnitt in Richtung der Synthesizeranschlüsse 34, 35 fort.

In einer nicht dargestellten Abwandlung des anhand Fig. 4 bis Fig. 7 erläuterten Ausführungsbeispieles sind die Ueberleitungsausnehmungen zwischen Schenkelausnehmungen 42, 43 in die den Reaktionskanälen 38 abgewandte Seite des ersten Aufnahmeteiles 25 eingebracht. Es versteht sich, dass die Ueberleitungsausnehmungen sowohl bei dem dargestellten Ausführungsbeispiel als auch bei der nicht dargestellten Abwandlung sich alternierend zwischen in Längsrichtung benachbarten Schenkelausnehmungen 42, 43 erstrecken können.

Somit sind bei dem anhand Fig. 4 bis Fig. 7 erläuterten Ausführungsbeispiel die Reaktionskanäle 38 bügelartig überspannende Zwischenkanäle 44 gebildet, die ausserhalb der Ebene verlaufen, in der die Reaktionskanäle 38 angeordnet sind, so dass eine dreidimensional mäanderartige Kanalanordnung gebildet ist, bei der auf einer Trägerfolie 3 als Trägermaterial allein in den von den oberflächenseitig von Reaktionskanälen 38 überdeckten Synthesebereichen Makromoleküle synthetisiert sind. Da die von Reaktionskanälen 38 überdeckten Synthesebereiche vollkommen voneinander separiert werden, ergibt sich bei diesem Ausführungsbeispiel der Vorteil, dass allein die gewünschten Sequenzen von Makromolekülen synthetisiert sind und nicht wie bei dem anhand Fig. 1 bis Fig. 3 erläuterten Ausführungsbeispiel in bei einigen Anwendungsfällen nachteiliger Weise in von Zwischenkanälen 16, 17 gegenüber den in Synthesebereichen vorliegenden Makromolekülen unterschiedlich aufgebaute Makromoleküle mit beispielsweise Sequenzlücken synthetisiert werden.

Fig. 8 zeigt in einer Schnittansicht in Querrichtung ein weiteres Ausführungsbeispiel einer Vorrichtung zum Synthetisieren von Makromolekülen, das über ein erstes Aufnahmeteil 50 und ein zweites Aufnahmeteil 51 verfügt. Das erste Aufnahmeteil 50 des in Fig. 8 dargestellten Ausführungsbeispieles 5 ist entsprechend dem anhand Fig. 2 und Fig. 3 erläuterten ersten Aufnahmeteil 1 mit einer Anordnung von in Querrichtung verlaufenden, zueinander parallelen ersten Reaktionskanälen 52 sowie sich endseitig an die ersten Reaktionskanäle 52 rechtwinklig anschliessenden ersten Schenkelausnehmungen 53 entsprechend dem anhand Fig. 5 erläuterten ersten Aufnahmeteil 25 versehen. Auf das erste Aufnahmeteil 50 ist in der Darstellung gemäss Fig. 8 auf der den ersten Reaktionskanälen 52 abgewandten Seite ein erstes Ueberleitungsteil 54 aufgelegt, das ähnlich dem anhand Fig. 7 erläuterten Ueberleitungsteil 29 aufgebaut ist, jedoch schiefwinklig angeordnete, schräg verlaufende erste Ueberleitungsausnehmungen 55 aufweist, um gegenüberliegende Enden benachbarter erster Reaktionskanäle 52 zusammen mit den ersten Schenkelausnehmungen 53 geschlossene erste Zwischenkanäle 56 bildend zu verbinden.

Weiterhin sind in das erste Ueberleitungsteil 54 sowie das erste Aufnahmeteil 50 miteinander fluchtende Durchsteckausnehmungen 57, 58 beispielsweise zum Durchführen von Gewindestangen oder Verbindungsschrauben eingebracht.

Das zweite Aufnahmeteil 51 ist im Querschnitt ähnlich dem anhand Fig. 4 und Fig. 5 erläuterten zweiten Aufnahmeteil 26 aufgebaut, verfügt jedoch wie das erste Aufnahmeteil 50 über parallel zueinander in Querrichtung ausgerichtete zweite Reaktionskanäle 59, die über zweite Schenkelausnehmungen 60 und über sowie in ein auf der dem ersten Aufnahmeteil 50 gegenüberliegenden Seite aufgelegtes zweites Ueberleitungsteil 61 engebrachte, zu dem zweiten Aufnahmeteil 51 offene zweite Ueberleitungsausnehmungen 62, die zusammen zweite Zwischenkanäle 63 bilden, mit ihren benachbart gegenüberliegenden Enden verbunden sind. Die ersten Reaktionskanäle 52 des ersten Aufnahmeteiles 50 sowie die zweiten Reaktionskanäle 59 des zweiten Aufnahmeteiles 51 sind dabei so relativ zueinander beabstandet und mit einer Reaktionskanalweite ausgebildet, dass die zweiten Reaktionskanäle 59 von durch zwischen den ersten Reaktionskanälen 52 ausgebildete erste Dichtabschnitte 64 und die ersten Reaktionskanäle 52 durch zwischen den zweiten Reaktionskanälen 59 ausgebildete zweite Dichtabschnitte 65 abdeckbar sind.

Weiterhin sind in dem zweiten Aufnahmeteil 51 sowie in dem zweiten Ueberleitungsteil 61 untereinander und mit den Durchsteckausnehmungen 57, 58 des ersten Ueberleitungsteiles 54 sowie des ersten Aufnahmeteiles 50 fluchtende Durchsteckausnehmungen 66, 67 eingebracht.

Die anhand Fig. 8 erläuterte Vorrichtung zeichnet sich dadurch aus, dass in effektiver Weise beidseitig eines Trägermateriales mit verhältnismässig wenig Versatzschritten zum Positionieren bei kompakten Aufgaben Makromoleküle synthetisierbar sind.

Fig. 9 zeigt in Draufsicht einen Abschnitt eines relativ flachen Aufnahmeteiles 68 einer Vorrichtung zum Synthetisieren von Makromolekülen gemäss der Erfindung, in das parallel äquidistant angeordnete Reaktionskanäle 69 eingebracht sind, wobei die Reaktionskanäle 69 bei vorzugsweise gleicher Länge in Querrichtung des Aufnahmeteiles 68 beziehungsweise in Längsrichtung der Reaktionskanäle 69 gegeneinander verschoben sind. An die Enden der Reaktionskanäle 59 schliessen sich in einer alternierenden Folge gleichseitig gelegene Enden von Reaktionskanälen 69 verbindende Zwischenkanäle 70, 71 an, die als geschlossene Leitungen ausserhalb der Ebene verlaufen, in der die Reaktionskanäle 69 angeordnet sind. Bei dem in Fig. 9 dargestellten Ausführungsbeispiel sind die Zwischenkanäle 70, 71 durch flexible Kunststoffleitungen gebildet, die gegenüber den durchzuleitenden Reagenzlösungen chemisch neutral sind.

Fig. 10 zeigt in Draufsicht einen Abschnitt eines weiteren Aufnahmeteiles 72 einer Vorrichtung zum Synthetisieren von Makromolekülen gemäss der Erfindung, das über parallel äquidistant angeordnete Reaktionskanäle 73 verfügt, die bei unterschiedlicher Länge vorzugsweise symmetrisch einer Mittelachse 74 des Aufnahmeteiles 72 angeordnet sind. An die Reaktionskanäle 73 schliessen sich in ähnlicher Weise wie bei dem anhand Fig. 9 erläuterten Ausführungsbeispiel in alternierender Folge endseitig mündende Zwischenkanäle 75, 76 an.

Bei positioniertem Versetzen der Reaktionskanäle 69, 73 der Aufnahmeteile 68, 72 in bezug auf ein Trägermaterial unter wiederholtem Ueberdecken der Synthesebereiche sind Makromoleküle synthetisierbar, die in einem überlappenden Mittenbereich der Reaktionskanäle 69, 73 einen jeweils gleichen Aufbau, in sich an die Mittenbereiche anschliessenden Randbereichen jedoch über eine definiert verkürzte Struktur verfügen.

Fig. 11 zeigt eine Draufsicht auf die Trägerfolie 3, bei der durch Aufbringen des ersten Aufnahmeteiles 1 gemäss dem anhand Fig. 1 bis Fig. 3 erläuterten Ausführungsbeispiel einer Vorrichtung zum Synthetisieren von Makromolekülen in einer ersten Position mit durch die Reaktionskanäle 15 überdeckte Synthesebereiche 77 sowie durch die ersten Zwischenkanäle 16 überdeckte Randreaktionsbereiche 78 und durch die zweiten Zwischenkanäle 17 überdeckte zweite Randreaktionsbereichen 79 umgrenzt dargestellt sind.

Fig. 12 zeigt die Anordnung gemäss Fig. 11 nach Durchleiten eines mit einem ersten Reagenz versehenen Lösungsmittels als erste Reagenzlösung durch die Reaktionskanäle 15 und die Zwischenkanäle 16, 17 in der ersten Position mit den Reaktionskanälen 15 und den Zwischenkanälen 16, 17, wobei die Reaktionskanäle 15 und Zwischenkanäle 16, 17 gegenüber der in Fig. 11 dargestellten Position um die Summe aus dem Reaktionskanalzwischenabstand 21 und der Reaktionskanalweite 24 versetzt sind. Mit einer von links oben nach rechts unten fallenden Schrägschraffur sind bei dem ersten Syntheseschritt in der ersten Position der Reaktionskanäle 15 und Zwischenkanäle 16, 17 auf der Trägerfolie 3 gebildete erste Monomere symbolisch dargestellt. Weiterhin zeigt Fig. 12 in einer unausgefüllten Umrandung in der zweiten Position den erstmalig durch einen Reaktionskanal 15 überdeckten Synthesebereich 77 und die erstmalig bedeckten Randreaktionsbereiche 78, 79.

In dieser zweiten Position der Trägerfolie 3 ist beispielsweise eine ein anderes Reagenz enthaltende zweite Reagenzlösung durch die sich mäanderförmig aneinanderreihende Abfolge von Reaktionskanälen 15 und Zwischenkanälen 16, 17 durchgeleitet, wobei sich in den zum zweiten Mal bedeckten Synthesebereichen 77 aus dem ersten Reagenz und dem zweiten Reagenz aufgebaute Dimere und in dem in der Darstellung gemäss Fig. 12 rechts liegenden, erstmalig von einem Reaktionskanal 15 überdeckten Synthesebereich 77 mittels des zweiten Reagenz zweite Monomere ausbilden, während in dem in der Darstellung gemäss Fig. 12 linken Synthesebereich 77 lediglich die in dem ersten Reaktionsschritt mittels des ersten Reagenz gebildeten ersten Monomere vorliegen.

Fig. 13 zeigt entsprechend den Darstellungen gemäss Fig. 11 und Fig. 12 Synthesebereiche 77 sowie Randreaktionsbereiche 78, 79 auf der Trägerfolie 3 nach vier Syntheseschritten, bei denen Reagenzlösungen mit vier verschiedenen Reagenzien bei vier zueinander jeweils um den Reaktionskanalzwischenabstand 21 und die Reaktionskanalweite 24 versetzten Positionierungen der Reaktionskanäle 15 sowie der Zwischenkanäle 16, 17 ausgebildet sind. Durch unterschiedliche Schraffuren sind der unterschiedliche Bildungsgrad der Makromoleküle in den Synthesebereichen 77 beziehungsweise den Randreaktionsbereichen 78, 79 bildhaft dargestellt. In den lediglich mit einer von links oben nach rechts unten fallenden Schrägschraffur ausgefüllten Synthesebereichen 77 und Randreaktionsbereichen 78, 79 sind nur Monomere mit dem in dem ersten Syntheseschritt durchgeleiteten ersten Reagenz gebildet. In den lediglich mit einer von links unten nach rechts oben steigenden Schrägschraffur ausgefüllten Randreaktionsbereichen 78, 79 sind Monomere mit dem bei dem zweiten Syntheseschritt durchgeleiteten zweiten Reagenz gebildet. In den durch einen in der Darstellung gemäss Fig. 13 lediglich mit einer von oben nach unten verlaufenden Querstrichelung ausgefüllten Randreaktionsbereiche 78, 79 sind in dem dritten Syntheseschritt mittels des durchgeleiteten dritten Reagenz dritte Monomere gebildet. In dem in der Darstellung gemäss Fig. 13 lediglich mit einer horizontalen Strichelung gefüllten, rechts gelegenen Synthesebereich 77 und einem Randreaktionsbereich 79 liegen bei dem vierten Syntheseschritt mittels des vierten Reagenz gebildete vierte Monomere vor.

In den übrigen Synthesebereichen 77 und Randreaktionsbereichen 78, 79 liegen in Abhängigkeit von ihrer vorangegangenen Ueberdeckung durch Reaktionskanäle 15 sowie Zwischenkanäle 16, 17 unterschiedlich ausgebildete Dimere, Trimere sowie Makromoleküle mit vier Struktureinheiten vor, wobei sich in der Darstellung gemäss Fig. 13 durch die Ueberlagerung der vorangehend beschriebenen vier unterschiedlichen Schraffuren für die durchgeleiteten vier Reagenzien erkennen lässt, welche Anzahl von Struktureinheiten vorliegt und welche Reagenzien bei der Bildung beteiligt waren.

Durch weiteres Versetzen der Trägerfolie 3 relativ zu den Reaktionskanälen 15 lassen sich sequenzhomologe Makromoleküle mit einer steigenden Anzahl von Struktureinheiten bilden, wobei zur Bildung von n Oligomeren der Kettenlänge m nur m + n - 1 Positionierungen und Syntheseschritte notwendig sind. Dabei ist es notwendig, dass die Anzahl an Reaktionskanälen 15, 38, 52, 59, 69, 73 wenigstens gleich der maximal zu synthetisierenden Kettenlänge ist.

Fig. 14 zeigt in einer anschaulich symbolhaften Darstellung eine Trägerfolie 3, auf die mit Ordnungszahlen 1 bis 26 spaltenweise gekennzeichnete Reihen von Makromolekülen mit durch einzelne Buchstaben repräsentierten Struktureinheiten aufgebaut worden sind. Dabei wurde ein Aufnahmeteil mit neun Reaktionskanälen eingesetzt, das in achtzehn Syntheseschritten unter Versatz der Reaktionskanäle in bezug auf die Trägerfolie bei exakter Ueberdeckung von Synthesebereichen versetzt worden ist. Bei den einzelnen Syntheseschritten wurden an jeweils einer Position der Reaktionskanäle nacheinander durch Buchstaben a, b, d, h, i, n, o, r, t, y, z repräsentierte Reagenzlösungen in einer das Wort "hybridizationarray" bildenden Abfolge durchgeleitet. An den in der Darstellung gemäss Fig. 14 spaltenweise durchnumerierten Positionen 8 bis 17 wurden die gewünschten vollständigen überlappenden zehn verschiedenen Sequenzen von aus neun Struktureinheiten aufgebauten Makromolekülen gebildet. An den mit Ziffern 1 bis 8 und 18 bis 26 gekennzeichneten Positionen wurden sequenzverkürzte Analoge einer von den Spalten 1 bis 8 jeweils um eins ansteigenden und von den Spalten 18 bis 26 jeweils um eins abnehmenden Anzahl von Struktureinheiten gebildet.

## Patentansprüche

1. Vorrichtung zum Synthetisieren von Makromolekülen insbesondere in sequenzhomologen Reihen mit wenigstens einem eine Anzahl von Reaktionskanälen (15, 38, 52, 59, 69, 73) aufweisenden Aufnahmeteil (1, 26, 50, 51, 68, 72) und mit einem Dichtabschnitte (64, 65) aufweisenden weiteren Aufnahmeteil (2, 25, 50, 51), mit denen die Reaktionskanäle (15, 38, 52, 59, 69, 73) abdeckbar sind, wobei zwischen den Aufnahmeteilen (1, 2, 25, 26, 50, 51 68, 72) ein in Synthesebereichen (77) von Reaktionskanälen (15, 38, 52, 59, 69, 73) überdeckbares Trägermaterial (3) in einer Anzahl von unterschiedlichen Anordnungen in bezug auf die Reaktionskanäle (15, 38, 52, 59, 69, 73) positionierbar ist, **dadurch gekennzeichnet**, dass Zwischenkanäle (16, 44, 56, 63, 70, 71, 75, 76) vorgesehen sind, über die eine Anzahl von Reaktionskanälen (15, 38, 52, 59, 69, 73) miteinander verbunden sind.

2. Vorrichtung nach Anspruch 1, dadurch gekennzeichnet, dass die Reaktionskanäle (15, 38, 52, 59, 69, 73) in einem Reaktionskanalzwischenabstand (21, 40) äquidistant angeordnet sind.

3. Vorrichtung nach Anspruch 2, dadurch gekennzeichnet, dass der Reaktionskanalzwischenabstand (21, 40) grösser als die Reaktionskanalweite (24, 41) ist.

4. Vorrichtung nach einem der Ansprüche 1 bis 3, dadurch gekennzeichnet, dass die Reaktionskanäle (15, 38, 52, 59, 69, 73) parallel ausgerichtet sind.

5. Vorrichtung nach einem der Ansprüche 1 bis 4, dadurch gekennzeichnet, dass die Zwischenkanäle (16, 17) in der Ebene (18) der Reaktionskanäle (15) angeordnet und zu einem Trägermaterial (3) offen sind.

6. Vorrichtung nach einem der Ansprüche 1 bis 4, dadurch gekennzeichnet, dass die Zwischenkanäle (44, 56, 63, 70, 71, 75, 76) sich als geschlossene Leitungen ausserhalb der Ebene (18, 39) erstrekken, in der die Reaktionskanäle (38, 52, 59, 69, 73) angeordnet sind.

7. Vorrichtung nach einem der Ansprüche 1 bis 6, dadurch gekennzeichnet, dass die Zwischenkanäle (16, 44, 56, 63, 70, 71, 75, 76) im Endbereich der Reaktionskanäle (15, 38, 52, 59, 69, 73) münden.

8. Vorrichtung nach einem der Ansprüche 1 bis 7, dadurch gekennzeichnet, dass sich die Reaktionskanäle (15, 52, 59, 69, 73) rechtwinklig zu der Versatzrichtung des Trägermateriales (3) erstrecken.

9. Vorrichtung nach einem der Ansprüche 1 bis 7, dadurch gekennzeichnet, dass sich die Reaktionskanäle (38) schiefwinklig zu der Versatzrichtung des Trägermateriales (3) erstrecken.

10. Vorrichtung nach einem der Ansprüche 1 bis 9, dadurch gekennzeichnet, dass die Reaktionskanäle (15, 38, 52, 59, 69) gleich lang sind.

11. Vorrichtung nach einem der Ansprüche 1 bis 9, dadurch gekennzeichnet, dass die Reaktionskanäle (73) eine unterschiedliche Länge aufweisen.

12. Vorrichtung nach einem der Ansprüche 1 bis 11, dadurch gekennzeichnet, dass die Reaktionskanäle (69, 73) in ihrer Längsrichtung um einen Teil ihrer Länge gegeneinander versetzt sind.

13. Vorrichtung nach einem der Ansprüche 1 bis 12, dadurch gekennzeichnet, dass die Aufnahmeteile (50, 51) relativ zueinander versetzt angeordnete Reaktionskanäle (52, 59) aufweisen, wobei zwischen den Reaktionskanälen (52, 59) eines Aufnahmeteiles (50, 51) Dichtabschnitte (64, 65) für die Reaktionskanäle (59, 52) des anderen Aufnahmeteiles (51, 50) ausgebildet sind.

14. Vorrichtung nach einem der Ansprüche 1 bis 13, dadurch gekennzeichnet, dass ein Aufnahmeteil (1, 26, 50) einen sich längs erstreckenden Führungsvorsprung (4, 27) und das andere Aufnahmeteil (2, 25, 51) eine zu dem Führungsvorsprung (4, 27) komplementäre Führungsausnehmung (5, 28) zur Führung eines sich in Längsrichtung erstreckenden Trägermateriales (3) aufweisen.

15. Vorrichtung nach einem der Ansprüche 1 bis 14, dadurch gekennzeichnet, dass Befestigungselemente (12, 33) zum lösbaren Aufeinanderpressen der Aufnahmeteile (1, 2, 25, 26, 50, 51, 68, 72) vorgesehen sind.

16. Vorrichtung nach einem der Ansprüche 1 bis 15, dadurch gekennzeichnet, dass unabhängig voneinander mit einer Reagenzlösung beaufschlagbare Gruppen von miteinander über Zwischenkanäle (16, 45, 56, 63, 70, 71, 75, 76) verbundenen Reaktionskanälen (15, 32, 52, 59, 69, 71) vorgesehen sind.

17. Verfahren zum Synthetisieren von Makromolekülen insbesondere in sequenzhomologen Reihen, bei dem über eine Anzahl von Reaktionskanälen (15, 38, 52, 59, 69, 73) Reaktionslösungen in Synthesebereichen (77) über ein Trägermaterial (3) geleitet werden, **dadurch gekennzeichnet**, dass miteinander über Zwischenkanäle (16, 44, 56, 63, 70, 71, 75, 76) in Verbindung stehende Reaktionskanäle (15, 38, 52, 59, 69, 73) und das Trägermaterial (3) bei verschiedenen Syntheseschritten in unterschiedlichen Relativpositionen Synthesebereiche (77) überdeckend angeordnet werden.

18. Verfahren nach Anspruch 17, dadurch gekennzeichnet, dass ein relativer Versatz von Reaktionskanälen (15, 38, 52, 59, 69, 73) in bezug auf das Trägermaterial (3) zu unmittelbar benachbarten Synthesebereichen (77) durchgeführt wird.

19. Verfahren nach Anspruch 17 oder Anspruch 18, dadurch gekennzeichnet, dass die Reaktionskanäle (15, 38, 52, 59, 69, 73) in bezug auf das Trägermaterial (3) bei allen Syntheseschritten in einer Richtung versetzt werden.

20. Verfahren nach einem der Ansprüche 17 bis 19, dadurch gekennzeichnet, dass die Anzahl der Syntheseschritte wenigstens der Anzahl der Reaktionskanäle (15, 38, 52, 59, 69, 73) entspricht.

**Claims**

1. A device for the synthesis of macromolecules, especially in sequentially homologous series, having at least one housing member (1, 26, 50, 51, 68, 72) comprising a number of reaction channels (15, 38, 52, 59, 69, 73) and having a further housing member (2, 25, 50, 51) comprising sealing portions (64, 65), with which the reaction channels (15, 38, 52, 59, 69, 73) can be covered over, in which device a carrier material (3), which can be covered in synthesis zones (77) by reaction channels (15, 38, 52, 59, 69, 73), can be positioned between the housing members (1, 2, 25, 26, 50, 51, 68, 72) in a number of different arrangements relative to the reaction channels (15, 38, 52, 59, 69, 73), **wherein** intermediate channels (16, 44, 56, 63, 70, 71, 75, 76) are provided by which a number of reaction channels (15, 38, 52, 59, 69, 73) are connected to one another.

2. A device according to claim 1, wherein the reaction channels (15, 38, 52, 59, 69, 73) are arranged equidistantly apart at a spacing (21, 40) between reaction channels.

3. A device according to claim 2, wherein the spacing (21, 40) between reaction channels is greater than the width (24, 41) of the reaction channels.

4. A device according to any one of claims 1 to 3, wherein the reaction channels (15, 38, 52, 59, 69, 73) are aligned in parallel.

5. A device according to any one of claims 1 to 4, wherein the intermediate channels (16, 17) are arranged in the plane (18) of the reaction channels (15) and are open towards a carrier material (3).

6. A device according to any one of claims 1 to 4, wherein the intermediate channels (44, 56, 63, 70, 71, 75, 76) extend in the form of closed conduits outside the plane (18, 39) in which the reaction channels (38, 52, 59, 69, 73) are arranged.

7. A device according to any one of claims 1 to 6, wherein the intermediate channels (16, 44, 56, 63, 70, 71, 75, 76) open into the end regions of the reaction channels (15, 38, 52, 59, 69, 73).

8. A device according to any one of claims 1 to 7, wherein the reaction channels (15, 52, 59, 69, 73) extend at right angles to the direction of displacement of the carrier material (3).

9. A device according to any one of claims 1 to 7, wherein the reaction channels (38) extend at an oblique angle relative to the direction of displacement of the carrier material (3).

10. A device according to any one of claims 1 to 9, wherein the reaction channels (15, 38, 52, 59, 69) are the same length.

11. A device according to any one of claims 1 to 9, wherein the reaction channels (73) have different lengths.

12. A device according to any one of claims 1 to 11, wherein the reaction channels (69, 73) are displaced with respect to one another in their longitudinal direction by a portion of their length.

13. A device according to any one of claims 1 to 12, wherein the housing members (50, 51) have reaction channels (52, 59) arranged displaced relative to one another, there being formed between the reaction channels (52, 59) of one housing member (50, 51) sealing portions (64, 65) for the reaction channels (59, 52) of the other housing member (51, 50).

14. A device according to any one of claims 1 to 13, wherein one housing member (1, 26, 50) has a guide projection (4, 27) that extends longitudinally and the other housing member (2, 25, 51) has a guide recess (5, 28) that is complementary to the guide projection (4, 27), for the purpose of guiding a carrier material (3) that extends in the longitudinal direction.

15. A device according to any one of claims 1 to 14, wherein fixing elements (12, 33) are provided for pressing the housing members (1, 2, 25, 26, 50, 51, 68, 72) against one another in a releasable manner.

16. A device according to any one of claims 1 to 15, wherein groups of reaction channels (15, 32, 52, 59, 69, 71) connected to one another by intermediate channels (16, 45, 56, 63, 70, 71, 75, 76) are provided, which groups are acted upon independently of one another by a reagent solution.

17. A process for the synthesis of macromolecules, especially in sequentially homologous series, in which by means of a number of reaction channels (15, 38, 52, 59, 69, 73) reaction solutions are conveyed in synthesis zones (77) over a carrier material (3), **wherein** reaction channels (15, 38, 52, 59, 69, 73), which are connected to one another by intermediate channels (16, 44, 56, 63, 70, 71, 75, 76), and the carrier material (3) are arranged in different relative positions in different synthesis steps to cover synthesis zones (77).

18. A process according to claim 17, wherein a relative displacement of reaction channels (15, 38, 52, 59, 69, 73) in relation to the carrier material (3) to directly adjacent synthesis zones (77) is carried out.

19. A process according to either claim 17 or claim 18, wherein the reaction channels (15, 38, 52, 59, 69, 73) are displaced in one direction relative to the carrier material (3) in all synthesis steps.

20. A process according to any one of claims 17 to 19, wherein the number of synthesis steps corresponds at least to the number of reaction channels (15, 38, 52, 59, 69, 73).

**Revendications**

1. Dispositif de synthèse de macromolécules, en particulier en séries de séquences homologues avec au moins une partie réceptrice (1, 26, 50, 51, 68, 72) présentant un certain nombre de canaux de réaction (15, 38, 52, 59, 69, 73) et avec une autre partie réceptrice (2, 25, 50, 51) présentant des sections d'étanchéité (64, 65), avec lesquelles les canaux de réaction (15, 38, 52, 59, 69, 73) peuvent être recouverts, où entre les parties réceptrices (1, 2, 25, 26, 50, 51, 68, 72) un matériau support (3) recouvrable dans les zones de synthèse (77) par les canaux de réaction (15, 38, 52, 59, 69, 73) peut être positionné en un certain nombre de dispositions différentes par rapport aux canaux de réaction (15, 38, 52, 59, 69, 73), caractérisé en ce que sont prévus des canaux intermédiaires (16, 44, 56, 63, 70, 71, 75, 76) par l'intermédiaire desquels un certain nombre de canaux de réaction (15, 38, 52, 59, 69, 73) sont reliés entre eux.

2. Dispositif selon la revendication 1, caractérisé en ce que les canaux de réaction (15, 38, 52, 69, 73) sont disposés de manière équidistante dans un intervalle entre les canaux de réaction (21, 40).

3. Dispositif selon la revendication 2, caractérisé en ce que l'intervalle entre les canaux de réaction (21, 40) est supérieur à la largeur d'un canal de réaction (24, 41).

4. Dispositif selon l'une des revendications 1 à 3, caractérisé en ce que les canaux de réaction (15, 38, 52, 59, 69, 73) sont disposés de façon parallèle.

5. Dispositif selon l'une des revendications 1 à 4, caractérisé en ce que les canaux intermédiaires (16, 17) sont disposés dans le plan (18) des canaux de réaction (15) et sont ouverts sur un support (3).

6. Dispositif selon l'une des revendications 1 à 4, caractérisé en ce que les canaux intermédiaires (44, 56, 63, 70, 71, 75, 76) s'étendent sous forme de canalisations fermées à l'extérieur du plan (18, 39) dans lequel les canaux de réaction (38, 52, 59, 69, 73) sont disposés.

7. Dispositif selon l'une des revendications 1 à 6, caractérisé en ce que les canaux intermédiaires (16, 44, 56, 63, 70, 71, 75, 76) débouchent dans la zone d'extrémité des canaux de réaction (15, 38, 52, 59, 69, 73).

8. Dispositif selon l'une des revendications 1 à 7, caractérisé en ce que les canaux de réaction (15, 52, 59, 69, 73) s'étendent à angle droit par rapport à la direction de décalage du support (3).

9. Dispositif selon l'une des revendications 1 à 7, caractérisé en ce que les canaux de réaction (38) s'étendent selon un angle oblique par rapport à la direction de décalage du support (3).

10. Dispositif selon l'une des revendications 1 à 9, caractérisé en ce que les canaux de réaction (15, 38, 52, 59, 69) sont de même longueur.

11. Dispositif selon l'une des revendications 1 à 9, caractérisé en ce que les canaux de réaction (73) présentent une longueur différente.

12. Dispositif selon l'une des revendications 1 à 11, caractérisé en ce que les canaux de réaction (69, 73) sont décalés les uns par rapport aux autres d'une partie de leur longueur dans leur direction longitudinale.

13. Dispositif selon l'une des revendications 1 à 12, caractérisé en ce que les parties réceptrices (50, 51) présentent des canaux de réaction (52, 59) disposés de manière décalée les uns relativement aux autres, et en ce qu'entre les canaux de réaction (52, 59) d'une partie réceptrice (50, 51) sont disposées des sections d'étanchéité (64, 65) pour les canaux de réaction (59, 52) de l'autre partie réceptrice (51, 50).

14. Dispositif selon l'une des revendications 1 à 13, caractérisé en ce qu'une partie réceptrice (1, 26, 50) présente une saillie de guidage (4, 27) s'étendant longitudinalement et en ce que l'autre partie réceptrice (2, 25, 51) présente un orifice de guidage (5, 28) complémentaire à la saillie de guidage (4, 27) afin de guider un support (3) s'étendant en direction longitudinale.

15. Dispositif selon l'une des revendications 1 à 14, caractérisé en ce que des éléments de fixation (12, 33) sont prévus pour presser l'une contre l'autre de façon amovible les parties réceptrices (1, 2, 25, 26, 50, 51, 68, 72).

16. Dispositif selon l'une des revendications 1 à 15, caractérisé en ce que sont prévus des groupes de canaux de réaction (15, 32, 52, 59, 69, 71), pouvant être alimentés indépendamment les uns des autres par une solution de réactif, reliés les uns aux autres par des canaux intermédiaires (16, 45, 56, 63, 70, 71, 75, 76).

17. Procédé de synthèse de macromolécules, en particulier en séries de séquences homologues, dans lequel, par l'intermédiaire d'un certain nombre de canaux de réaction (15, 38, 52, 59, 69, 73) des solutions réactionnelles sont conduites dans des zones de synthèse (77) par l'intermédiaire d'un support (3), caractérisé en ce que les canaux de réaction (15, 38, 52, 59, 69, 73) en liaison les uns avec les autres au moyen des canaux intermédiaires (16, 44, 56, 63, 70, 71, 75, 76) et le support (3) sont disposés de façon à recouvrir des zones de synthèse (77) dans des positions relatives différentes lors d'étapes de synthèse différentes.

18. Procédé selon la revendication 17, caractérisé en ce qu'on réalise un décalage relatif des canaux de réaction (15, 38, 52, 59, 69, 73) par rapport au support (3) dans des zones de synthèse (77) immédiatement voisines les unes des autres.

19. Procédé selon la revendication 17 ou la revendication 18, caractérisé en ce que les canaux de réaction (15, 38, 52, 59, 69, 73) sont décalés dans une direction par rapport au support (3) lors de toutes les étapes de synthèse.

20. Procédé selon l'une des revendications 17 à 19, caractérisé en ce que le nombre des étapes de synthèse correspond au moins au nombre des canaux de réaction (15, 38, 52, 59, 69, 73).

**Fig. 1**

**Fig. 2**

EP 0 880 405 B1

Fig. 3

EP 0 880 405 B1

Fig. 4

EP 0 880 405 B1

**Fig. 5**

**Fig. 6**

**Fig. 7**

17

Fig. 8

EP 0 880 405 B1

69    69    68

71

70

# Fig. 9

72    76

76    73

74

75

75

# Fig. 10

**Fig. 11**

Fig. 12

**Fig. 13**

EP 0 880 405 B1

EP 0 880 405 B1

```
  1     3     5     7     9     11    13    15    17    19    21    23    25
     2     4     6     8     10    12    14    16    18    20    22    24    26
```

**3**

```
h h h h h h h h h y b r i d i z a t i o n a r r a y
    y y y y y y y y b r i d i z a t i o n a r r a y
      b b b b b b b r i d i z a t i o n a r r a y
          r r r r r r i d i z a t i o n a r r a y
            i i i i i d i z a t i o n a r r a y
              d d d d i z a t i o n a r r a y
                i i i z a t i o n a r r a y
                  z z a t i o n a r r a y
                    a t i o n a r r a y
```

# Fig.14